# EUROPEAN PATENT SPECIFICATION

(11) **EP 2 773 962 B1**
(45) Date of publication and mention of the grant of the patent: **14.09.2016**
(21) Application number: 12845749.6
(22) Date of filing: 01.11.2012
(51) Int. Cl.: G01N 33/574

(54) **ASSAY FOR PREDICTIVE BIOMARKERS OF ANTI-ESTROGEN EFFICACY**
TEST FÜR PRÄDIKTIVE BIOMARKER DER WIRKSAMKEIT GEGEN ÖSTROGEN
DOSAGE POUR BIOMARQUEURS DE PRÉDICTION D'EFFICACITÉ ANTI- STROGÈNE

(30) Priority: 04.11.2011 US 201161555635 P
(43) Date of publication of application: 10.09.2014
(73) Proprietor: Invivis SAS, 91400 Orsay (FR)
(72) Inventor: GILLES, Erard, Bridgewater, NJ 08807 (US)
(74) Representative: Hally, Anna-Louise
(86) International application number: PCT/US2012/062989
(87) International publication number: WO 2013/067136

(56) References cited:
- WO-A1-2005/028681
- US-A- 5 202 931
- US-A- 5 202 931
- US-A1- 2010 167 945
- KOCANOVA SILVIA ET AL: "Ligands specify estrogen receptor alpha nuclear localization and degradation", BMC CELL BIOLOGY, BIOMED CENTRAL, LONDON, GB, vol. 11, no. 1, 10 December 2010 (2010-12-10), page 98, XP021086062, ISSN: 1471-2121, DOI: 10.1186/1471-2121-11-98
- SUZANNE E WARDELL ET AL: "The turnover of estrogen receptor by the selective estrogen receptor degrader (SERD) fulvestrant is a saturable process that is not required for antagonist efficacy", BIOCHEMICAL PHARMACOLOGY, ELSEVIER, US, vol. 82, no. 2, 31 March 2011 (2011-03-31) , pages 122-130, XP028227385, ISSN: 0006-2952, DOI: 10.1016/J.BCP.2011.03.031 [retrieved on 2011-04-09]
- Y. ISHII ET AL: "Bortezomib Enhances the Efficacy of Fulvestrant by Amplifying the Aggregation of the Estrogen Receptor, Which Leads to a Proapoptotic Unfolded Protein Response", CLINICAL CANCER RESEARCH, vol. 17, no. 8, 3 February 2011 (2011-02-03), pages 2292-2300, XP055199030, ISSN: 1078-0432, DOI: 10.1158/1078-0432.CCR-10-1745
- KOCANOVA S. ET AL.: '"Ligands specify estrogen receptor alpha nuclear localization and degradation"' BMC CELL BIOLOGY 01 January 2010, pages 1 - 13, XP021086062

## Description

### TECHNICAL FIELD

The present invention relates to biomarkers associated with anti-estrogen sensitivity in cancers, methods for detecting and quantitating the biomarkers, and methods for treating cancer patients that exhibit the biomarkers.

### BACKGROUND

Prognostic and predictive biomarkers are routinely used in clinical management of patients with breast cancer, and their assessment has become mandatory as a basis for therapeutic decision-making. One such biomarker is the estrogen receptor (ER), which is a nuclear transcription factor activated by estrogen to regulate growth and differentiation of normal breast epithelial cells. Estrogen also stimulates growth of tumor cells which express ERα, and ER expression in tumors is a good prognostic factor for breast cancer patients. However, ERα expression is also highly predictive of tumor sensitivity to therapeutic intervention with anti-estrogens. Anti-estrogens include direct receptor antagonists (e.g., tamoxifen and falzodex) and aromatase inhibitors (e.g., anastrozole, letrozole and exemestane). Anti-estrogens represent the best treatment currently available for breast cancer in the adjuvant setting. Unfortunately, at the present time there is no method for accurately predicting the efficacy of such adjuvant therapies for a particular tumor in a particular patient, as treatment failure is not recognizable until relapse. It is known, however, that in the metastatic setting and when ER is expressed by tumors, only 50% of these cancers respond to anti-estrogen treatment. This indicates clearly that, while absence of receptors predicts absence of effect, only a subset of cancers expressing ER will benefit from anti-estrogen treatment.

ERα expression in tumors is evaluated in immunohistochemical assays using labeled antibodies targeting the receptor, which results in visible staining. Typically, formalin-fixed paraffin-embedded tumor specimens are evaluated under direct microscopic visualization, and the number of stained cells is quantitated as a percentage of total cells. There is significant variation in positivity by this method, and ERα-expressing tumors may contain from almost 0 to nearly 100% positive cells. Although there is no real correlation between the likelihood of clinical response to hormonal therapies and the level of ERα expression, it is remarkable that even tumors expressing very low levels (e.g., 1-10% positive cells) may show a significant response, whereas ERα-negative tumors are essentially completely unresponsive. For this reason, a threshold of 1% positive cells has generally been used as the definition of ERα positivity.

The immunohistochemical assays described above provide only a quantitation of the estrogen receptor itself. That is, they do not take into account whether or not it is bound to its ligand in DNA, the Estrogen Receptor Element (ERE), or whether is has any functional role. Current immunohistochemical assays therefore do not provide any information on whether or not the estrogen receptor that is detected is also biologically or transcriptionally activated. Like other steroid receptors, estrogen receptors are predominantly present in the nucleus of the cell. In the absence of binding to ERE the estrogen receptor is seen as a diffuse nuclear staining in immunohistofluorescence assays. Academic studies in experimental biology have reported that nuclear receptors such as estrogen receptor form nuclear aggregates or foci in the presence of ligand which are visible by microscopy. These subnuclear structures may also be referred to as speckles and the nuclei containing them as hyperspecked nuclei. Upon ligand binding the receptor moves into the subnuclear aggregate structure to activate transcription of a wide variety of genes.

The most commonly used methods in studies visualizing the formation of estrogen receptor foci have employed cell lines transiently transfected with green fluorescent protein (GFP)-tagged ERα. One such commercially available assay is the ERα Redistribution® Assay from Thermo Scientific (Biolmage Products, Lafayette, CO). This assay is designed to assay compounds for their ability to modulate accumulation of ERα in nuclear foci using GFP to monitor translocation. Nuclear foci are detected and analyzed by an image analysis algorithm, revealing ligand-regulated movement of the transfected estrogen receptors into subnuclear foci. Nuclear distribution of endogenous receptors has also been detected in cell lines using immunoflourescence but there have been very few studies examining nuclear receptor distribution in animal or human primary tissues. While these types of assays are useful for elucidating possible biologic mechanisms of estrogen receptor foci formation, they do not provide any information concerning the process, how it occurs, or its relevance to disease in naturally-occurring cancers. Reports of focal ER reactions observed on immunohistochemical staining of tissue section or fine needle aspiration smears are actually an artifact of inadequate fixation or heterogeneity of staining in the cancer tissue. *See, for example*, M. Nadji et al. (2005) Am. J. Clin. Pathol. 123: 21-27*.* Such focal staining of heterogenous cancer tissue is observable at low magnification and, in contrast to subnuclear ER foci, does not relate to a specific molecular structure.

In fact, basic academic research in this field shows that the available model systems are not representative of the clinical variability of cancer. As of 2005 more than 140 human breast cancer cell lines have been developed, but only about one third of them express estrogen receptors and/or estrogen receptors. About 50 mouse cell lines have been developed and only a few express estrogen receptor or estrogen receptor. In contrast, in the clinical setting 75% of breast cancer patients have tumors which are positive for hormone receptors. These statistics illustrate the inadequacy of *in vitro* model systems with respect to developing improved methods for selecting appropriate cancer treatment for individual breast cancer patients.

In naturally occurring cancers ERα bound to ligand in activated foci potentially activates or inactivates thousands of genes. It has therefore been suggested that the immunohistochemical assay currently used to identify ERα positive breast cancer patients and to determine whether anti-estrogen treatment is appropriate could be improved by developing assays which analyze multigene prognostic and predictive signatures. This approach is based on the assumption that response to hormonal therapies is biologically too complex to be accurately predicted by measuring expression of a single gene (i.e., the expression of only ERα). (D. C. Allred, Modern Pathology (2010) 23, S52-S59). However, assaying thousands of genes to determine a predictive profile or attempting to pinpoint those that are most relevant is a costly and very long-term strategy. In addition, because the goal of estrogen receptor assays is primarily to determine which cancers are likely to respond to anti-estrogen treatment, and which are not, the status of individual genes (i.e., activated or suppressed) is unlikely to be relevant to the clinical goal of antagonizing the collective effects of estrogen on the biology of cancer. (see e.g. WO2005/028681)

There is therefore a need for improved estrogen receptor assays, including ERα assays, which can more accurately and sensitively identify breast cancer patients that are most likely to respond to anti-estrogen therapy. Such assays better define which patients will benefit from anti-estrogen treatment. There is also a clear need for methods which improve the ability to identify estrogen receptor positive tumors are likely to be most precisely targeted by anti-estrogen therapy. Better targeting assists in avoiding unnecessary treatment and in developing more relevant treatment strategies. For example, if anti-estrogens were known in advance to be ineffective in a particular tumor (particularly in a tumor that is ERα positive by conventional methods), unnecessary treatment would be avoided and other treatment options could be initiated more quickly with better expected outcomes. The ability to predict anti-estrogen efficacy in a particular tumor would also permit early selection of synergistic treatment combinations such as an mTOR inhibitor (e.g., everolimus).

There is also a need for assay methods which more accurately and sensitively predict the efficacy of anti-estrogen treatment in individual breast cancer patients because there is presently no means for knowing that treatment has failed until the patient has relapsed. The ability to instead predict potential anti-estrogen treatment failure at the time of diagnosis provides great value to the decision to undertake the current 5-year program of anti-estrogen adjuvant therapy. Early knowledge of potential anti-estrogen treatment failure can therefore avoid unnecessarily putting the patient at risk for side effects of treatment, such as endometrial cancer, and support early decisions to select more appropriate treatment strategies at a time when the chances of success are better.

The present invention satisfies these needs. In contrast to the assays of the prior art, such as the Thermo Scientific Estrogen Receptor alpha Redistribution^{®} Assay, the present invention provides analysis of ER foci in primary tumor tissue, irrespective of the presence of an ER ligand or a drug. In one aspect, the exemplary methods described herein relate to the presence of ER foci in the nuclei of cells in naturally-occurring tumors indicating an anomaly that can be used to predict the efficacy in that patient of an anti-estrogen that has ER antagonist properties. In another aspect, the characterization of constitutively activated ER in the clinic has now been found to be a new and useful indicator of tumors and cancers that are susceptible to treatment with anti-estrogens.

### SUMMARY

Applicants have determined that the foregoing needs in the art for improved estrogen receptor (including ERα) assays employing primary breast cancer tissue samples can be met by immunohistochemical or cytological assays that distinguish cancers in which the estrogen receptor is biologically activated from those in which estrogen receptor is present but biologically inactive. These assays detect the presence of ER foci or aggregates in the nuclei of cancer cells (biologically, transcriptionally active ER) as an indication of ER-positive status of the tumor, as opposed to ER-negative status indicated by diffuse nuclear staining (biologically inactive ER) and/or no nuclear staining. The presence of biologically active ER provides an indication that the estrogen receptor that is present is engaged in the biology of the tumor and is therefore an appropriate target for anti-estrogen therapy. In contrast, diffuse nuclear staining of estrogen receptors indicates that, although the tumor would be considered ER-positive by conventional methods, it is unlikely to be sensitive to anti-estrogen therapy due to the biologically inactive status of the receptor.

The present invention therefore provides a method for identification of a subset of ER-positive tumors most likely to benefit from treatment with anti-estrogens such as tamoxifen, falzodex, anastrozole, letrozole, and exemestane. Formation of estrogen receptor foci has been studied in homogeneous, artificial experimental models. However, each naturally-occurring tumor is different and each naturally-occurring tumor is heterogeneous. The clinical significance of distinguishing tumor cells expressing estrogen receptor in biologically activated form (i.e., bound to ligand in foci or aggregates) from tumor cells expressing estrogen receptor in biologically inactive form (not bound to ligand and in a diffuse nuclear distribution) was not previously recognized. Expression of biologically active (i.e., transcriptionally active) estrogen receptors, visualized as foci within the nuclei of cancer cells, provides a potential target for intervention by anti-estrogens and disruption of estrogen-stimulated pathways of tumor cell growth. That is, in such tumor cells anti-estrogens can potentially inactivate ligand activated or other aberrantly activated ER. In contrast, expression of transcriptionally inactive ER, visualized as diffuse nuclear staining on immunohistochemical or cytological evaluation, would indicate that in spite of the presence of estrogen receptor in tumor cells it is unlikely to be an appropriate target for anti-estrogen intervention. This approach would provide a rational framework for understanding the therapeutic efficacy of anti-estrogen in breast cancers, because most female breast cancer patients are postmenopausal and no physiological impact of anti-estrogen would be expected. The identification of those tumors that express aberrantly activated ER by the pathological biologic background would provide a direct and plausible rationale for treatment.

ER foci in tumor cells can therefore serve as a biomarker for selection of an appropriate treatment, including the decision whether or not to employ anti-estrogens in adjuvant therapy. Recognition of this biomarker allows development of an assay which can serve as an indicator of the likelihood that a breast cancer patient will benefit from therapy with anti-estrogens.

In a first embodiment, the diagnostic assay is a method for identifying patients having tumors which express activated ER (i.e., activated ER foci or "AEF"). These patients are more likely to benefit from treatment with an anti-estrogen than patients that do not express activated ER (typically seen as diffuse nuclear staining). Inactivation of the AEF by an anti-estrogen may occur by any of a variety of mechanism, including dissociation of the foci and inhibition of activation of the foci without substantially altering their structure. Patients that do not express activated ER foci (AEF) may include those that are estrogen receptor negative by the conventional assay, or those that are estrogen receptor positive by the conventional assay. Any tumor which exhibits AEF is believed to be a candidate for treatment with such anti-estrogens, including breast cancer.

In a second embodiment, the invention relates to an *in vitro* method for identifying a tumor treatable with an AEF-active drug, comprising:
a) exposing cancer cells or a tissue specimen containing cancer cells obtained from a patient to an anti-estrogen receptor antibody under conditions appropriate for binding of the antibody to estrogen receptors in nuclei of the cancer cells; and
b) detecting presence or absence of focal binding of the antibody to estrogen receptors in the nuclei;
wherein the presence of focal binding indicates sensitivity of the tumor to treatment with the AEF-active drug and the absence of focal binding indicates lack of sensitivity of the tumor to treatment with the AEF-active drug.

Further disclosed is treating a patient positive for focal binding of the antibody with an estrogen receptor antagonist. Yet further disclosed is treating a patient positive for focal binding of the antibody with an aromatase inhibitor.

In yet another embodiment, focal binding of the antibody is detected in the absence of diffuse binding of the antibody in the nuclei.

In yet another embodiment, focal binding of the antibody is detected in addition to diffuse binding of the antibody in the nuclei.

In yet another embodiment, the cancer cells are breast cancer cells or the primary tumor tissue specimen is a breast cancer specimen.

In yet another embodiment, presence or absence of focal binding is detected by fluorescence.

In yet another embodiment, presence or absence of focal binding is detected in a colorimetric reaction, such as an enzymatic reaction.

In yet another embodiment, the method further comprises detecting presence or absence of focal binding in a quantitative or semi-quantitative manner.

Disclosed is a method for treating a tumor, comprising:
a) exposing cancer cells or a tissue specimen containing cancer cells obtained from a patient to an anti-estrogen receptor antibody under conditions appropriate for binding of the antibody to estrogen receptors in nuclei of the cancer cells;
b) detecting presence or absence of focal binding of the antibody to estrogen receptors in the nuclei; and
c) treating the patient with an AEF-active anti-estrogen if focal binding is present.

In yet another embodiment, the invention relates to use of an AEF-active anti-estrogen for treating a tumor, following identification of the presence of focal binding of anti-estrogen receptor antibody to estrogen receptors in cancer cell nuclei.

In any of the foregoing embodiments, presence of focal binding may mean 1-100%, 5-100%, 25-100% or 50-100% of cancer cell nuclei exhibiting focal binding.

In any of the foregoing embodiments, when focal binding is present, the intensity or density of such focal binding may be quantitated.

In any of the foregoing embodiments the AEF-active anti-estrogen may be a receptor antagonist or an aromatase inhibitor, or the tumor of interest may be breast cancer.

In yet another embodiment, the invention provides an *in vitro* method for screening an antitumor drug or antitumor drug candidate for AEF inactivating activity which comprises:
a) providing cancer cells or a tumor tissue specimen containing cancer cells, wherein the cancer cells express a baseline degree of focal distribution of AEF and the AEF are detectably stained with an anti-estrogen receptor antibody;
b) exposing the cancer cells or the tumor tissue specimen to the antitumor drug or antitumor drug candidate; and
c) detecting a decrease in degree of focal distribution of the AEF relative to baseline as an indication of AEF inactivating activity of the antitumor drug or drug candidate, or detecting no substantial decrease in the degree of focal distribution of the AEF relative to baseline as an indication of lack of AEF inactivating activity of the antitumor drug.

In certain embodiments of methods for screening an antitumor drug or antitumor drug candidate for AEF inactivating activity, the baseline level of AEF may be expressed as the percentage of cells exhibiting AEF or the average number of AEF per cell. A decrease in detectable staining of AEF after exposure to the drug or drug candidate would then be expressed as a reduced percentage of cells expressing AEF or a reduced average number of AEF per cell relative to baseline. Alternatively, a reduction in the average size of AEF could be used to indicate AEF inactivating activity of the drug or drug candidate.

In other embodiments of the methods for screening an antitumor drug or antitumor drug candidate for AEF inactivating activity, the cancer cells may be provided as a cancer cell line that expresses a baseline level of AEF. Alternatively, the tumor tissue specimen may be a primary tissue specimen or tissue derived from a biopsy.

In any of the foregoing embodiments, the estrogen receptor detected may be ERα and the anti-estrogen receptor antibody used may be anti-ERα.

### DESCRIPTION OF THE DRAWINGS

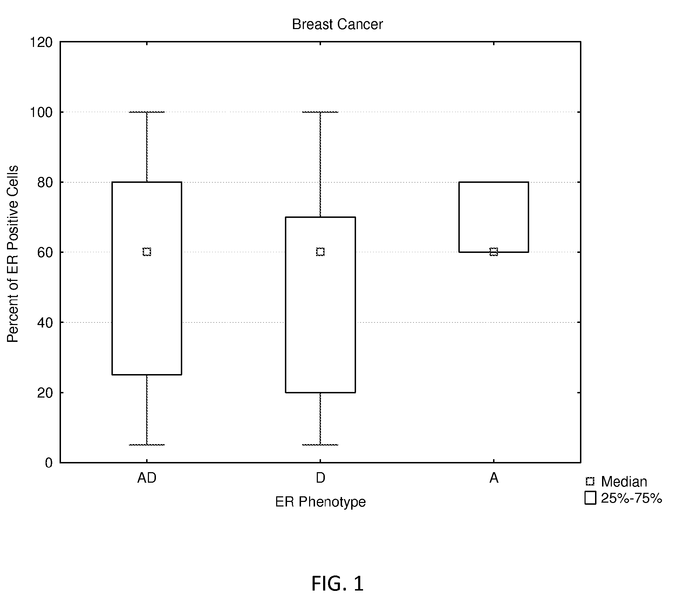

Fig. 1 illustrates the results of Example 5. The plot shows the correlation of percent ER positivity of breast cancer biopsies using conventional methods (y axis) with AEF status using the methods of the invention (x axis).

### DETAILED DESCRIPTION

As used herein, the terms "cancer cells" and "tumor cells" are generally interchangeable and refer to malignant cells which may be present in a solid tumor or which may be circulating in the blood. The solid tumor may be a primary tumor or a metastatic tumor, and any circulating cancer cells may be derived from either solid tumor type. Analysis of solid tumors for purposes of the invention (i.e., histological analysis) may be performed using a primary biopsy specimen. Alternatively, cancer or tumor cells for analysis (i.e., cytological analysis) may be obtained by fine needle aspiration of a solid tumor as well as by separation from blood.

As used herein, the phrases "treating a tumor," "treatment of a tumor" and the like mean to inhibit the replication of tumor cells, inhibit the spread of the tumor, decrease tumor size, lessen or reduce the number of tumor cells in the body, or ameliorate or alleviate the symptoms of the disease caused by the tumor. Tumors include cancers. The treatment is considered therapeutic if there is a decrease in mortality and/or morbidity, or there is a decrease in disease burden as may be manifested by reduced numbers of tumor cells in the body or decreased tumor size.

As used herein, the term "estrogen receptor" includes the dimerized estrogen receptor, the ERα receptor and the ERβ receptor.

As used herein, the term "AEF-active anti-estrogen" and its equivalents refer to an anti-estrogen drug which exhibits an ability to inactivate, dissolve or dissociate activated ER foci (AEF) in the nuclei of cells, indicating that its mechanism of action is via the ER activation pathway of the cell. These terms are intended to include the dimerized estrogen receptor as well as the ERα receptor and the ERβ receptor

The terms "AEF-positive", "ER foci positive", "activated ER", "ERs in a functional state" and the like refer to the presence of estrogen receptor aggregates in the nuclei of cells. These terms are intended to include the dimerized estrogen receptor as well as the ERα receptor and the ERβ receptor. The terms include pathologically activated ER, i.e., ER that may not be activated by the usual physiological agonists such as estradiol or tamoxifen. The identification of AEF implies that activation is through an abnormal mechanism.

The term "degree of focal distribution" refers to the relative number of AEF-positive cells vs. total cells in a sample. The degree of focal distribution can be determined quantitatively or qualitatively. In one example, the degree of focal distribution is expressed as a percentage of AEF-positive cells, i.e., the percentage of cells that contain estrogen receptor aggregates (quantitative). In an alternative example, the degree of focal distribution may be expressed in relative terms describing the number of cells that contain estrogen receptor aggregates, such as "few" or "many" (qualitative).

For example, the use of a colorimetric, enzymatic, or radiolabeled ligand such as anti-estrogen receptor antibody, can be used to bind to estrogen receptors in cell nuclei. The degree of focal distribution can be determined quantitatively, for example, by determining the number of cells having color intensity, fluorescence, or radioactivity emitted by the labeled antibody which is associated with AEF rather than a diffuse staining pattern. The degree of focal distribution can be compared to a control sample which does not contain AEF-positive cells or which has a degree of focal distribution that is deemed below a lower threshold using a light microscope at an appropriate magnification or techniques including, but not limited to, DNA microarray, protein profiling, radiolabeling, or other surrogates for measuring ER foci.

The term "diffuse pattern" refers to a finely granular pattern which is indicative of the absence of focal distribution.

The term "estrogen" refers to a natural or synthetic estrogenic substance that mimics some or all of the actions of estradiol, also referred to as estrogen receptor modulators (ERM) or selective estrogen receptor modulators (SERM).

The term "anti-estrogen" refers to a substance that inhibits the formation, transport, or action of, or which inactivates progestational agents, including, but not limited to, fulvestrant, Tamoxifen, Toremifene, and aromatase inhibitors such as Letrozole, Anastrozole, Vorozole, Exemestane, Foremestane, and Atemestane. A ERM or SERM may have some anti-estrogen properties, and may be considered an anti-estrogen or an estrogendepending on the context of use.

An "AEF-active drug" is a drug that dissociates AEF or otherwise reduces the number, size or staining intensity of AEF in cell nuclei. In one example, an AEF-active drug causes the conversion of an A or AD staining pattern into a D staining pattern in the cell. AEF-active drugs include anti-estrogens and estrogen receptor antagonists as well as drugs that exhibit activity against AEF but to not operate by an anti-hormonal mechanism.

The term "antibody" or "antibodies" refers to a protein which is capable of specifically binding to an antigen and includes any substance, or group of substances, which has a specific binding affinity for the antigen to which it is directed, with little or no binding affinity for other substances. Generally, the term "antibody" includes polyclonal antibodies, monoclonal antibodies, antibodies derived from humans or animals, humanized antibodies (e.g., non-binding portions derived from a human, binding portions derived from an animal) and fragments thereof.

The terms "anti-ERα antibody" and "anti-ERβ antibody" refer to antibodies directed to the α and β isoforms of the estrogen receptor, respectively. "Anti-ER antibody" refers generically to an antibody capable of binding ER. Specific antibodies suitable for use in accordance with aspects herein include, but are not limited to CONFIRM SP1 anti-ER monoclonal antibodies available from Ventana Medical Systems (Tucson, AZ) and NOVOCASTRA anti-ERα and anti-ERβ monoclonal antibodies available from Leica Biosystems (Buffalo Grove, IL).

In one aspect, the invention provides a method of inhibiting the growth of a tumor susceptible to growth inhibition by anti-estrogens by determining the degree of focal binding of anti-ER antibody in nuclei of cells in a cell sample or tissue obtained from a patient and suspected of containing tumor cells. If the degree of focal distribution is greater than about 5%, for example from about 5% to 100%, 25-100% or 50-100%, an anti-estrogen is administered to the patient to inhibit growth of the tumor. Potentially useful anti-estrogens include fulvestrant ((7R,8R,9S,13S,14S,17S)-13-methyl-7-[9-(4,4,5,5,5-pentafluoropentylsulfinyl)nonyl]-6,7,8,9,11,12,14,15,16,17-decahydrocyclopenta[a]phenanthrene-3,17-diol), Tamoxifen (2-[4-[(Z)-1,2-diphenylbut-1-enyl]phenoxy]-N,N-dimethylethanamine), Toremifene (2-[4-[(Z)-4-chloro-1,2-diphenylbut-1-enyl]phenoxy]-N,N-dimethylethanamine), aromatase inhibitors such as Letrozole (4-[(4-cyanophenyl)-(1,2,4-triazol-1-yl)methyl]benzonitrile), Anastrozole (2-[3-(2-cyanopropan-2-yl)-5-(1,2,4-tuazol-1-ylmethyl)phenyl]-2-methylpropanenitrile), Vorozole (6-[(4-chlorophenyl)-(1,2,4-triazol-1-yl)methyl]-1-methylbenzotiiazole), Exemestane ((8R,9S,10R,13S,14S)-10,13-dimethyl-6-methylidene-7,8,9,11,12,14,15,16-octahydrocyclopenta[a]phenanthrene-3,17-dione), Foremestane ((8R,9S,10R,13S,14S)-4-hydroxy-10,13-dimethyl-2,6,7,8,9,11,12,14,15,16-decahydro-1H-cyclopenta[a]phenanthrene-3,17-dione), and Atemestane ((8R,9S,10S,13S,14S)-1,10,13-trimethyl-7,8,9,11,12,14,15,16-octahydro-6H-cyclopenta[a]phenanthrene-3,17-dione)), and combinations thereof.

In one aspect, the invention relates to a method for identifying a tumor treatable with an AEF-active drug, comprising:
a) exposing cancer cells or a tumor tissue specimen containing cancer cells obtained from a patient to an anti-estrogen receptor antibody under conditions appropriate for binding of the antibody to estrogen receptors in nuclei of the cancer cells; and
b) detecting presence or absence of focal binding of the antibody to estrogen receptors in the nuclei; wherein the presence of focal binding indicates sensitivity of the tumor to treatment with the AEF-active drug and the absence of focal binding indicates lack of sensitivity of the tumor to treatment with the AEF-activedrug.

The foregoing assay for focal ER binding provides a more sensitive and predictive test than currently-used conventional ER assays, and focal ER binding can be identified in patients classified in conventional ER assays as ER-negative as well as those that are conventionally ER-positive. Patients classified in conventional ER assays as ER-negative as well as those that are conventionally ER-positive may test positive for focal ER nuclear binding and therefore be considered candidates for treatment with anti-estrogens. The absence of ER foci in patients conventionally tested as ER-positive would explain the apparently anomalous result that anti-estrogens are ineffective in some of these patients. The assay method of the invention therefore makes hormonal treatment an efficacious choice for a greater number of cancer patients.

The cancer cells analyzed in any of the assays for focal binding may be contained in a specimen of tumor tissue taken directly from a patient. These specimens are typically referred to as primary biopsies, and may be derived from either primary or metastatic solid tumors (a histological analysis). Alternatively, the cancer cells analyzed in any of the assays for focal binding may be individual cancer cells or small clusters of cancer cells obtained, for example, by needle aspiration of a tumor or by separation of cancer cells from blood (a cytological analysis). Cytological analysis has several advantages, including being less invasive for the patient and it providing an analysis of the relevant cellular compartment without interference from surrounding tissue architecture.

Certain immunohistochemical methods suitable for use in the invention are described by M. Nadji, et al. (Am. J. Clin. Pathol. (2005) 123:21-27) and D. C. Allred (Modern Pathology (2010) 23:S52-S59). By way of example, primary tumor biopsy tissue specimens for analysis may be prepared as paraffin sections of the cancer tissue as is known in the art for conventional ER assays. If paraffin sections are used, the paraffin is first melted by heating the slides, and dewaxed with xylene. Slides are then rehydrated in decreasing grades of ethanol and exposed to an antibody, preferably a monoclonal antibody, which specifically binds to ERα, ERβ, or both. Binding of the antibody is then detected using any one of the methods known in the art for detection of antibody binding. As ERα is a commonly used biomarker for breast cancer, antibodies which specifically bind to ERα may be most appropriate for the methods of the invention.

Certain cytological methods suitable for use in the invention include immunocytochemical methods applied to fine needle aspiration materials, for example as described by N. H. Hafez, et al. (J. Egyptian Nat. Cancer Inst. (2010) 22:217-225). Aspiration cytology slides may be fixed in alcohol (e.g., 95% isopropyl alcohol, 10 min. to 18 hrs.) and stained to visualize estrogen receptor. Fixed slides may be exposed to a primary antibody specific for the ER, preferably a monoclonal antibody, which binds to ERα, ERβ, or both. Binding of the antibody may be detected using any one of the methods known in the art for detection of antibody binding.

One appropriate method for detection of binding of an antibody to its target is a colorimetric assay, typically an enzymatic colorimetric assay. One such method employs peroxidase to produce a colored stain visible under the light microscope. Endogenous peroxidase in the tissue specimen is blocked using hydrogen peroxide and endogenous biotin is blocked using a biotin-blocking reagent prior to incubation with the antibody or antibodies. In one example, binding of primary antibody is followed by biotinylated secondary antibody targeting the primary antibody. Binding of the secondary antibody is then detected using avidin or streptavidin conjugated to peroxidase, typically horse radish peroxidase (HRP). The conjugate is added to bind the enzyme to the antibody-target complex. ER is visualized by enzymatic conversion of the chromogenic substrate 3,3'-Diaminobenzidine (DAB) to a brown stain at the site of ER localization. If the primary antibody is a mouse antibody, it is subsequently bound to a biotinylated anti-mouse immunoglobulin. The cytological or tissue specimen may be counterstained with fast green to increase visibility of the peroxidase stain.

Alternatively, a fluorescence method may be used to detect antibody binding to ER-α, EAR-β or both. In this case, a fluorescently-labeled primary antibody may be bound to the ER target and detected directly under a fluorescence microscope. However, a method employing binding of an unlabeled primary antibody to the ER followed by binding a fluorescently-labeled secondary (e.g., anti-mouse immunoglobulin) antibody to the primary antibody may reduce non-specific fluorescence. Any fluorescent label known for use in immunocytological or immunohistochemical assays may be used in the methods of the invention, for example FITC.

Both monoclonal and polyclonal antibodies may be useful in the present methods. A non-exhaustive list of suitable monoclonal antibodies is available commercially from Santa Cruz Biotechnology, Inc., including both anti-ERα and anti-β antibodies (http://www.scbt.com/table-estrogen_receptor.html).

Binding of the antibody to ER is typically detected by observation of the stained slide under a light microscope or fluorescence microscope as appropriate. Magnification is typically about 40X or 50X; however, to improve sensitivity for detection of AEF it may be desirable to evaluate the slides at about 80X or 100X to facilitate study of subnuclear structures. The magnification can be adjusted as necessary, as is known in the art, to more clearly identify the various ER phenotypes described herein.

Samples that are apparently negative by microscopy may be evaluated by flow cytometry to detect positivity that is below the threshold of light or fluorescence microscopy. If flow cytometry indicates rare positive cells, high magnification microscopy (100X-400X, or 400X-900X, e.g., 800X) may be used to study subnuclear structures and identify AEF. However, if the positive cells detected by flow cytometry are too rare to be reliably detected by microscopy for analysis of AEF, a fluorescence-activated cell sorter (FACS) can be used to separate positive cells from the cells in suspension based on their fluorescence. As positive cells are concentrated but not damaged by this process, the reliability and probability of successfully visualizing AEF on subsequent microscopic evaluation is substantially increased.

The presence or absence of AEF in individual tumor cell nuclei may be detected visually under a light or fluorescence microscope, or by any other appropriate means, such as fluorescence or colorimetric measurements. Typically, visual means for detection will be used. The results of staining may be quantitated by simply noting presence or absence of AEF, or by counting the number or percentage of positive cells. Alternatively, specific characteristics of the staining may be quantitated. For example, detection may include notation of whether or not focal binding in the form of AEF is accompanied by diffuse nuclear staining, quantitation of positive cells by number or percentage, and/or quantitation of intensity or number/density of AEF. Relative size of AEF may also be used to characterize, classify and quantitate. Quantitation of AEF density may be determined as the average number of foci/cell, or using an arbitrary scale (e.g., "few", "moderate" or "many"). Intensity may similarly be determined using an arbitrary scale, e.g., low/medium/high or a numerical scale such as 1-5. Typically, the results of the analysis of the patient's tumor tissue will be compared to positive and/or negative controls.

A cytological or tumor tissue specimen is judged as AEF-positive when the degree of focal distribution is 1-100%, 5-100%, 25-100% or 50-100% of cells in the specimen exhibit AEF. As therapeutic efficacy of an AEF-active drug or anti-estrogen may also be correlated with the intensity of AEF staining or with the number, size or density of AEF, these parameters may also be used to determine the sensitivity of the tumor to treatment with the AEF-active drug or anti-estrogen. In general, it is anticipated that sensitivity of the tumor to treatment with AEF-active drugs or anti-estrogens will increase with the degree of focal distribution (e.g., increasing number or percentage of positive cells, increasing intensity of AEF and/or increasing number or size of AEF) in the cells of the cytological or tumor tissue specimen.

The above methods for determining the sensitivity of a tumor to AEF-active drugs or anti-estrogens may be either manual (e.g., visual detection using a fluorescence microscope) or they may be automated or semi-automated using methods for rapid scanning, detection and quantitation of colorimetrically- or fluorescently-labeled cytological or tissue specimens. For example, a fully automated scanning and analysis system may be developed and used in the present invention. In contrast to the InScape® ER/PR quantitative immunohistochemistry (IHC) system, which requires manual selection of specific regions to be analyzed, the present system for scanning and analysis of AEF in cell nuclei will be designed to provide automated whole-specimen scanning and analysis of the antigen-specific immunohistochemistry stained specimen. Image recognition will create a digital image of the entire stained tissue section. An antigen-specific computer algorithm will then analyze the results of the digital image representing the whole specimen. For use in the methods of the invention, the software will distinguish foci from diffuse background staining in the nucleus, and measure fluorescence intensity and size of foci on a cell-by-cell or cluster-by-cluster basis, repeating the process for each cell or cluster over the entire specimen. These automated methods will result in improved accuracy by performing a function that is not possible manually, with reduced cost. Full automation will also make the test accessible to non-expert medical centers.

The present methods for determining the sensitivity of a tumor to AEF-active drugs or anti-estrogens allow medical practitioners to identify a subset of tumor patients that are more likely to benefit from such treatment. That is, by first classifying the tumor as AEF-positive or AEF-negative in the diagnostic method, the medical practitioner is able to select the most appropriate treatment for the patient. Accordingly, in another aspect the disclosure also provides a method for treating a tumor in a patient, comprising:
a) exposing cancer cells or a tissue specimen containing cancer cells obtained from the patient to an anti-estrogen receptor antibody under conditions appropriate for binding of the antibody to estrogen receptors in nuclei of the cancer cells;
b) detecting presence or absence of focal binding of the antibody to estrogen receptors in the nuclei; and
c) treating the patient with an AEF-active drug if focal binding is present.

The details of the detection of the degree of focal distribution (including presence or absence of focal binding of the antibody to estrogen receptors) in the nuclei of cells are discussed above. The decision whether to treat the patient based on the results of the diagnostic assay will be based on the number/percentage, intensity, size and/or density of AEF when they are present. A decision to treat the patient with an AEF-active drug or anti-estrogen may be made when the degree of focal distribution in the diagnostic assay is 1-100%, 5-100%, 25-100% or 50-100% of cancer cell nuclei. Generally, it is anticipated that the efficacy of treatment with an AEF-active drug or anti-estrogen will increase with increasing number or percentage of positive cells, increasing intensity of AEF and/or increasing number or size of AEF in the cells of the tumor tissue specimen or cancer cell sample. Based on these parameters the medical practitioner may also determine the dosing, timing and length of treatment. Accordingly, in another embodiment the invention relates to use of an AEF-active anti-estrogen for treating an AEF-positive tumor.

The tumor to be identified or treated according to the above methods may include any cancerous or non-cancerous tumor in which AEF occur, and in which the presence of AEF can be determined. The tumor tissue or cancer cells for analysis or treatment may be selected from the group consisting of breast, prostate, ovarian, endometrial, lung, and uterine tissue or cells. Such cancers particularly include breast cancer.

The AEF-active drug or anti-estrogen of the foregoing treatment methods may be any drug having the ability to inactivate AEF (for example by dissolving or dissociating the aggregates). Such drugs include ER antagonists and/or aromatase inhibitors, but others with the ability to inactivate AEF as the mechanism of action are also included for use. In certain aspects, the anti-estrogen is selected from the group consisting of fulvestrant, Tamoxifen, Toremifene, or aromatase inhibitors such as Letrozole, Anastrozole, Vorozole, Exemestane, Foremestane, and Atemestane, and combinations thereof.

In yet another aspect, the AEF-active drug or anti-estrogen is administered to a patient having an AEF positive tumor in an amount from 10 to about 200 mg per day depending upon the potency, bioavailability, and safety profile of the selected anti-estrogen or combination of anti-estrogens. Without being bound by theory, it is believed that by identifying patients with tumors that are more susceptible to treatment with anti-estrogens, a lower dose of the anti-estrogen may be used, resulting in a lower risk of toxic side effects. Thus, a lower dosage range of about 10 to about 200 mg can be used for patients exhibiting greater than 5% focal binding of AEF. In one aspect, highly AEF positive classification of ER in a tumor could result in a different dosing regimen than classification as both AEF positive and diffusely staining cells, due to a higher degree of activation in the highly AEF positive tumor. In contrast, a predominantly diffuse staining pattern (indicating unactivated ER) would indicate that treatment with an AEF-active drug or anti-estrogen treatment is not warranted.

In yet another aspect, the invention provides methods for screening antitumor drugs and antitumor drug candidates for the ability to inactivate AEF. These methods are useful to identify additional AEF-active drugs, including anti-estrogens, which may be candidates for use in treating of AEF-positive tumors according to the methods of the invention. Accordingly, the method for screening an antitumor drug or antitumor drug candidate for AEF inactivating activity comprises:
a) providing cancer cells or a tumor tissue specimen containing cancer cells, wherein the cancer cells express a baseline degree of focal distribution of AEF and the AEF are detectably stained with an anti-estrogen receptor antibody;
b) exposing the cells or tumor tissue specimen to the antitumor drug or antitumor drug candidate; and
c) detecting a decrease in degree of focal distribution of the AEF relative to the baseline degree of focal distribution of the AEF as an indication of AEF inactivating activity of the antitumor drug or drug candidate, or detecting no substantial decrease in degree of focal distribution of the AEF relative to the baseline degree of focal distribution of the AEF as an indication of lack of AEF inactivating activity of the antitumor drug or drug candidate.

In an alternative embodiment of the foregoing screening method, two AEF expressing tumor tissue specimens or two samples of AEF-expressing cancer cells from the same tumor can be used for comparison. In this embodiment, both tumor tissue specimens or cancer cell samples are detectably stained for AEF with an anti-estrogen receptor antibody, then one of the specimens or samples is exposed to the antitumor drug or antitumor drug candidate and the other is not. If the staining of the AEF in the treated specimen or sample is decreased compared to the untreated specimen or sample, the antitumor drug or antitumor drug candidate, the antitumor drug or antitumor drug candidate has AEF inactivating activity. Conversely, if the staining of the AEF in the treated specimen or sample is not decreased compared to the untreated specimen or sample, the antitumor drug or antitumor drug candidate, the antitumor drug or antitumor drug candidate does not have AEF inactivating activity. The foregoing screening method will provide further understanding of the mechanism of action of known anti-estrogens and anti-estrogens yet to be discovered. Those which are AEF-active (i.e., have AEF inactivating activity) will likely be useful in treating tumors in patient populations identified as having AEF-positive tumors using the methods of the invention. Examples of anti-estrogens that may be screened as AEF-active include any of the anti-estrogens approved for use by regulatory authorities and any of the unapproved anti-estrogens in development.

If an antitumor drug is negative for AEF activity in the screening method described above, the lack of ability to inactivate AEF can be interpreted as an indication that the antitumor drug may be effective in combination therapy with a an AEF-active anti-estrogen due to complementarity of the different mechanisms of action. For example, an AEF-active anti-estrogen may be used in combination with additional hormonal treatment that does not act by an AEF inactivation mechanism (e.g., anti-progestins) to achieve improved therapeutic efficacy as compared to either agent alone. Alternatively, an AEF-active anti-estrogen may be used in combination with one or more conventional chemotherapeutic agents which are negative for AEF activity in the screening assay to achieve improved therapeutic efficacy as compared to either agent alone (e.g., everolimus, trastuzumab, TM1-D, anti-HER2 drugs, bevacizumab, or chemorapy with agents such as paclitaxel, docetaxel, taxanes, doxorubicin, liposomal doxorubicin, pegylated liposomal doxorubicin, anthracyclines, anthracenediones, carboplatin, cisplatin, 5-FU, gemcitabine and cyclophosphamide). For example, everolimus is an mTor inhibitor that is indicated in combination with an aromatase inhibitor and may, in the future, be indicated based on ERF.

In yet another aspect, detecting the presence of focal distribution of the antibody to AEF in the nuclei may be used as an indication that the tumor of a patient previously treated with an antitumor drug, which has become resistant to that drug, is still sensitive to an AEF-inactivating anti-estrogen. In one aspect, the method can be adapted to determine whether chemoresistance of a tumor resulting from previous chemotherapy can be reversed by treatment with an AEF-inactivating anti-estrogen. Reversal of such chemoresistance may be based on the different mechanisms of action of the previous chemotherapy and the AEF-inactivating anti-estrogen.

### Example 1

Tumor specimens from patients with breast cancer (invasive ductal carcinoma) and endometrial cancer were selected from the archives of Oscar Lambret Cancer Center (Lille, France), anatomical pathological department. Patients had previously provided consent for the use of their tissues for research purposes. Samples of breast or endometrial tumor tissues which had been fixed in 4% formalin fixative and embedded in paraffin were obtained.

Immunohistochemistry (IHC) was performed on 3-4 µm sections of the archival breast or endometrial tumor tissues. The sections were deparaffinized, hydrated and washed in working buffer (0.05 mol/L Tris/HCl, 0.15 mol/L NaCl, 0.05% Tween 20, pH 7.6, Dako, Denmark, code S3006). Antigen retrieval was carried out with the Dako Target Retrieval Solution (modified citrate buffer, pH 6.1, Dako, Denmark, code S1699) in a water bath at 98°C for 20 min. Then, the sections were covered with the Dako Peroxydase Block solution to block endogenous peroxides at room temperature (RT) for 5 min (Dako EnVision® +/HRP Mouse (DAB+) Kit, Dako, Denmark, code K4007), washed and incubated with the primary antibodies at the appropriate optimal dilutions at RT for 60 min in a humidified chamber (Table 1). Following a 5-min. wash with working buffer, the Dako Labelled Polymer (Dako EnVision® +/HRP Mouse (DAB+) Kit, Dako, Denmark, code K4007) was used for the detection of the primary antibody binding at RT for 30 min. Chromogen (DAB) was then used with Substrate-Batch at room temperature for 5-10 min and the sections were lightly counterstained with Gill's hematoxylin.

Negative controls were obtained by substitution of the primary antibodies with isotype control mouse IgG1 (Table 1) or with antibody diluent alone (wash buffer negative control) in the immunohistochemical staining procedure.

**Table 1. Antibodies used for immunohistochemistry**

| **Anti-ERα** | | | **Monoclonal Mouse** | | **IgG1** | | **Clone 6F11** | | AbD serotec **MCA1799** (1ml) |
|---|---|---|---|---|---|---|---|---|---|
| | Recommandations: | | | | | | | Lot 270412 | |
| | unmasking : citrate buffer | | | | | | | Exp. NP | |
| | Dilution : 1/40 to 1/80 | | | | | | | Conc. between 10 and | |
| | | | | | | | | 50mg/L | |
| http://www.abdserotec.com/product/6f11-anti-estrogen-receptor-alpha-antibody-mca1799.html | | | | | | | | | |

| **Anti-ER** | | **Monoclonal** Rabbit | | **IgG** | | **Clone SP1** | | Thermo Scientific **MA1-39540** (1ml) | |
|---|---|---|---|---|---|---|---|---|---|
| | **Recommandations:** | | | | | | Lot 1574651 | | |
| | Unmasking : citrate buffer, boiling for 10 min | | | | | | Exp. NC | | |
| , cooling for 20 min | | | | | | | Conc. NC | | |
| | Dilution : 1/200, 30 minutes at ambient | | | | | | | | |
| temperature | | | | | | | | | |
| | http://www.pierce-antibodies.com/Estrogen-Receptor-antibody-clone-SP1-Monoclonal-MA139540.html | | | | | | | | |

Immunohistochemistry analysis was performed using a Zeiss Axioscope microscope, equipped with an Imaging Model ROHS digital camera. Immunoreactive signals were classified as unequivocal brown labeling of tumor cell nuclei. The intensity of labeling was defined as 0 for negative, + for weak, ++ for moderate and +++ for strong.

### Example 2

Breast cancer samples were analyzed with two different antibodies. 15 samples were processed for further immunohistofluorescence (IHF) analysis.

Immunohistofluorescence was performed using a Zeiss fluorescent microscope equipped with a CCD camera and Smart Capture software, specific for capture of fluorescent images. IHF was performed on 3-4 µm sections of the archival breast tumor tissues. The sections were deparaffinized, hydrated and washed in working buffer (0.05 mol/L Tris/HCl, 0.15 mol/L NaCl, 0.05% Tween 20, pH 7.6, Dako, Denmark, code S3006). Antigen retrieval was carried out with the Dako Target Retrieval Solution (modified citrate buffer, pH 6.1, Dako, Denmark, code S1699) in a water bath at 98°C for 20 min. Then, the sections were incubated with the primary antibodies at the appropriate optimal dilutions at RT for 60 min in a black humidified chamber (Table 2). Following a 5-minute wash with working buffer, appropriate secondary antibody conjugated to Alexa Fluor 488 was used for the detection of the primary antibody binding at RT for 30 min (Anti-mouse IgG (H+L), F(ab')2, Cell Signaling, USA, code 4408S, dilution 1:1000 ; Anti-rabbit IgG (H+L), F(ab')2, Cell Signaling, USA, code 4412S, dilution 1:1000). All slides were then washed and covers lipped using Vectashield® HardSet Mounting Medium (Vector Labs, USA, code H-1400) and stored refrigerate in the dark until analysis, to preserve fluorescence. Negative controls were obtained by substitution of the primary antibodies with isotype control mouse IgG1 or rabbit serum (see IHC table) or with antibody diluent alone (wash buffer negative control) in the immunohistofluorescence staining procedure.

Thereafter, a larger sample was analyzed in IHC with the Anti-ER Alpha antibody.

70 breast cancers and 20 endometrial cancer samples were processed. For each labeled tumor sample, positive focal distribution was defined as the percentage of labeled tumor cells in the entire tumor tissue, excluding necrotic areas.

Two basic staining patterns were observed upon staining of tissue samples with anti-ER antibodies using IHC. The first pattern was a brown, finely granular, and diffuse pattern referred to herein as "D". The second pattern was a mottled, clumped pattern representing a positive focal binding pattern referred to herein as "A". The same two basic patters were observed in samples processed using IHF. Both the diffuse D pattern and the mottled, clumped, focal binding pattern A observed with IHF were similar to the IHC result. The diffuse D and focal binding A patterns were similar to the results obtained in gene-engineered cells that express a fluorescent receptor when no steroid or no steroid-agonist is present (Arnett-Manfield et Al, 2004, 1C Control, 1D, and 1E) and in normal human endometrial tissue and in endometrial cancer (Arnett-Manfield et Al, 2004, 1A, 1B, 1C, 1D, 1E, 1F).

The active A pattern observed in formalin fixed, paraffin embedded tumor tissue may differ from images obtained in fresh cells. This is expected because formalin-fixation and paraffin embedding tissue will result in changes to the cellular contents, thereby resulting in a different pattern of ER. Another difference relative to the research publications which utilized IHF, is related to the method. In the research setting, a confocal microscope (i.e. using two laser beams) provides high resolution and 3D images;. The IHC pattern results from a chemical reaction that modifies the cellular content. In contrast for IHC, a traditional wide-field microscope is used for reading the standard tumor slices (e.g., 4 microns). The IHC technique described results in some loss of resolution.

The IHF technique is less chemically aggressive for tumor tissues, in that it does not alter the microscopic cellular architecture. IHF requires specialized, equipment, a pathologist experienced with the technique, and is much more time-consuming. IHF cannot be easily coupled with other pathology analyses such as standard histology that requires formalin-fixed paraffin embedded tissues. Thus, in one aspect, IHC may be used as a routine pathological laboratory procedure. In the developed IHC technique used herein, 4 micrometer tissue sections (a commonly used thickness for routine clinical analysis) were used for all analyses.

Thus, two basic patterns were found: a diffuse ER nuclear staining "D" indicating an absence of activated ERs, and heterogeneous staining "A" where aggregates, indicating AEF, can be recognized within the nucleus of the cells. ER foci are visibly larger than elements of a diffuse D pattern, which are substantially smaller.

### Example 3

In total, three categories or phenotypes of ER staining have been identified which are observed at higher magnification (e.g., 80X). In contrast, standard magnification (200-400X) is used for ER status determination in conventional IHC.

Categories (observed at high magnification) are:
D : Diffuse Staining, no ER foci (i.e., AEF)
AD : Area containing both A and D cells, or having a heterogeneous distribution of ER foci (AEF) with smaller sizes than observed in the A phenotype
A Large foci (AEF) distributed in a heterogeneous manner.

This classification (D, AD, and A) was evaluated on 90 cases (70 breast cancer and 20 endometrial cancer tissue samples).

Breast cancer samples (61 cases) were analyzed for ER expression using conventional techniques. Of the 70 total breast cancer cases, seven were ER negative for all antibodies, and two had missing data. Table 2 and Table 3 show the results of the conventional assay for ER in breast cancer and endometrial cancer tumor cells, followed by the respective AEF profile:

**Table 2**

| Breast Cancer Tumor Cells Positive for Indicated Antibody | | | | |
|---|---|---|---|---|
| ***In Percentages*** | Number of Cases | Mean | Min | Max |
| Anti-ER | 61 | 51% | 5% | 100% |

| | | | | |
|---|---|---|---|---|
| Focal Distribution (Breast Cancer) D: 34/61 (56%) AD: 24/61 (39%) A : 3/61 (5%) | | | | |

**Table 3**

| Endometrial Cancer Cells Positive for Indicated Antibody | | | | |
|---|---|---|---|---|
| 25 Cases (14 Cases Negative for ER Antibody) | | | | |
| ***In Percentages*** | Number of ER Positive Cases | Mean | Min | Max |
| Anti-ER | 11 | 36% | 5% | 15% |

| | | | | |
|---|---|---|---|---|
| Focal Distribution (Endometrial Cancer) D Type: 4/11 AD Type: 7/11 | | | | |

The section below describes the frequencies of A, AD, D patterns and N (negative, no ER staining) in the tumor samples previously evaluated for ER positivity using conventional methods. All cases were analyzed at high magnification (100X). Some breast cancer cases were not evaluable. Conventional IHC methods to determine ER cannot provide information on staining pattern because they only indicate the presence or absence of hormone receptors. Expression of the activated ER patterns (e.g., A and AD) is heterogeneous in tumors and across different samples, which is a characteristic of cancers. In contrast, the D phenotype is homogeneous, a pattern consistent with a lack of ER biologic function.

### Example 4

The ER positivity rate for 61 breast cancers using conventional methods for analysis was 51% with a standard deviation of 31%. These tumors would be considered ER positive and treatment with anti-estrogen would be considered to be indicated. ER positivity was also scored using a scale of from 1 = weak to 3 = strong, to measure the quality of the staining. However, it was found that the degree of staining was not related to activated ER status as shown in the following table:

**Table 4**

| | | | | AER | | |
|---|---|---|---|---|---|---|
| | | D | AD | | A | Total |
| ER Score | 1 | 8 | 11 | | 1 | 20 |
| | 2 | 17 | 11 | | 1 | 29 |
| | 3 | 9 | 2 | | 1 | 12 |
| | Total | 34 | 24 | | 3 | 61 |

### Example 5

The plot of FIG. 1 shows the percent of breast cancer samples positive for ER by conventional methods (percentage of tumor cells expressing ER, y axis) compared to the three binding patterns (A, AD, and D) for the same cancers determined by methods according to the invention (x axis). The tumor is considered ER positive if more than 1% of cells are positive. Usually, however, clinicians use 10% positive cells as the cut-off for the decision to administer anti-estrogen treatment. As shown in FIG. 1, tumors having an activated ER status (AEF - A or AD) and tumors having an unactivated ER status (D) both have a similar ER positivity rate as determined by conventional methods, indicating that conventional ER positivity does not predict the AEF status of the tumor cell. That is, the median value for all three AEF-based phenotypes correlates with about 60% ER positivity by conventional methods. These results support the conclusion that a positive ER status determined by conventional methods does not correlate with the presence of AEF.

### Example 6

Table 5 shows the percentage of ER Positive tumor cells in breast cancer biopsies with the AEF type: A, AD or D. Fig. 1 shows the percent positivity for each AEF type. It is shown that the A, D, or AD type is not associated with the conventional ER rate. Thus, the AEF is not predicted by the standard ER staining rate.

**Table 5 Percentage of Cells expressing an AEF pattern**

| ER Positive Cells Percent | AEF Type |
|---|---|
| 5 | AD |
| 5 | D |
| 20 | D |
| 60 | AD |
| 60 | D |
| 70 | D |
| 100 | D |
| 100 | AD |
| 90 | AD |
| 5 | AD |
| 20 | AD |
| 5 | D |
| 5 | D |
| 80 | AD |
| 60 | AD |
| 40 | AD |
| 20 | D |
| 80 | AD |
| 70 | D |
| 70 | D |
| 90 | AD |
| 70 | D |
| 70 | D |
| 60 | AD |
| 90 | D |
| 5 | D |
| 5 | D |
| 70 | D |
| 5 | D |
| 50 | D |
| 60 | D |
| 70 | AD |
| 80 | D |
| 40 | D |
| 70 | D |
| 60 | A |
| 40 | D |
| 60 | A |
| 80 | A |
| 30 | AD |
| 5 | AD |
| 80 | D |
| 5 | AD |
| 80 | D |
| 30 | D |
| 40 | D |
| 30 | AD |
| 80 | D |
| 90 | D |
| 90 | AD |
| 30 | D |
| 5 | AD |
| 5 | D |
| 60 | D |
| 30 | AD |
| 90 | D |
| 5 | D |
| 50 | D |
| 90 | AD |
| | AD |
| 30 | AD |
| | AD |
| 5 | D |

For endometrial cancers, eleven cases were positive for ER using conventional techniques. The average percentage of ER positive tumor cells was 0.6%, with a minimum of 0.05% and a maximum of 0.15%. Four of these tumors exhibited a D pattern upon staining for AEF, and seven exhibited the A or AD staining pattern. The four cancers with the D phenotype had less than 10% ER-positive cells. A-phenotype cancers had up to 15% positive cells with the antibody used. AEF is present in a minority of cases. The use of anti-estrogens in endometrial cancer is hampered with inconsistent data. Identifying the right subset of patients is likely to give more reliable and interpretable results and to assist in identifying the right patients who would benefit for a specific hormotherapy. Patients with receptors and AEF might benefit from agents disrupting AEF, those with inactivated but present ER might benefit from other endocrine agents.

### Example 7

Tumor cell lines are grown in monolayers, with a culture media adapted for each line, after amplification for 3 days. Each cell line is cultured in either Normal FBS or charcoal-Stripped FBS. Each serum condition is exposed to growth factors or hormones: either Vehicle, RPMI, Oestradiol 10nM Progesterone 30 ng/ml (i.e. 100 nM), EGF 10 ng/ml or FGF 2.5 ng/ml. Each experimental setting is supplemented with Vehicle (DMSO), and an anti-estrogen test substance 100 nM or 1 µM. Cell viability is assessed with trypan blue exclusion.

**The following cell lines are tested:**

| **Cell line** | **Type** | **Specie** | **Origin** |
|---|---|---|---|
| **BT**-**474** | Mammary tumor | Human | ATCC^{a} |
| **CAMA**-**1** | Mammary tumor | Human | ATCC^{a} |
| **EVSA**-**T** | Mammary tumor | Human | ATCC^{a} |
| **HCC**-**1954** | Mammary tumor | Human | ATCC^{a} |
| **MCF**-**7** | Mammary tumor | Human | ATCC^{a} |
| **MDA**-**MB-231** | Mammary tumor | Human | ATCC^{a} |
| **T**-**47D** | Mammary tumor | Human | ATCC^{a} |
| **ZR**-**75**-**1** | Mammary tumor | Human | ATCC^{a} |
| **Ishikawa** | Uterine Endometrial Cancer | Human | ATCC^{a} |
| **HEC-1-A** | Uterine Endometrial Cancer | Human | ATCC^{a} |

For cytoblocks, WB and cytotoxic assays, cells are incubated in 75 cm² flasks, 25 cm² flasks and 6-well plates, respectively, and treated with hormones and anti-estrogen test substance at T = 0 hours and re-treated similarly at T = 4 days. For cytoblocks and WB, the cells are collected at T = 6 hours, T = 4 days and T = 7 days. For cytotoxic assays, the cells are observed at T = 7 days. The experiment is performed twice with duplicate conditions within each experiment.

The *in vitro* cytotoxic activity of the test substance is revealed by an MTS assay. For each condition of treatment, the ratio between Growth Factor or hormone treated only and control (RPMI) conditions is calculated to show the effect of hormone on growth. The ratio of anti-estrogen test substance + Growth Factor over Growth Factor (or Control) is calculated to estimate anti-estrogen effect of the reference condition.

Cytoblocks of formalin-fixed paraffin embedded cells are produced at time = 6 hours, 4 days and 7 days. Test blocks are evaluated with Hematin-Eosine Saffran and IHC for PR receptors. If the coloration is too intense for nuclear analysis, separated HES slides and ER immunochemistry w/o background may be performed.

IHC is performed on 3-4 µm sections. The sections are deparaffinized, hydrated and washed in working buffer. Antigen retrieval is carried out. Then, the sections are covered with the Dako Peroxydase Block solution to block endogenous peroxides at room temperature (RT) for 5 min, washed and incubated with the primary antibodies at the appropriate optimal dilutions at RT for 60 min in a humidified chamber. Following a 5-min. wash with working buffer, the Dako Labelled Polymer is used for the detection of the primary antibody binding at RT for 30 min. Chromogen (DAB) is then carried out with Substrate-Batch at RT for 5-10 min and the sections are lightly counterstained with Gill's hematoxylin n°2. Negative controls are obtained by substitution of the primary antibodies with isotype control mouse IgG1 (Table 1) or with antibody diluent alone (wash buffer negative control) in the immunohistochemichal staining procedure. Immunohistochemichal analysis is performed using a Zeiss Axioscope microscope, equipped with a digital camera or scanner.

Immunoreactive signals are analyzed at x40 magnification and classified as unequivocal brown labelling of tumor cell nuclei. The intensity of labelling iss defined as 0 for negative, + for weak, ++ for moderate and +++ for strong. The percentage of tumor positive cells is calculated.

IHC is conducted for ER-α, ER-β and ER. Subsequently, subnuclear morphology is analyzed at x 100 magnification using a microscope and photos are scanned at high resolution with XY (ref). As described previously subnuclear morphology is described as diffuse when a diffuse staining is observed or A when a mottled pattern is observed. The sample is determined as AD when cells of D or A types are mixed.

It is found that when cells express the D staining pattern there is no sensitivity to the anti-estrogen test substance, even if the cells are ER-positive by conventional methods. All cells that are sensitive to the anti-estrogen test substance are either A or AD staining pattern. It is also observed that cell convert from A or AD pattern to D pattern even when they are growing with various stimuli other than hormones. This indicates a biological effect which does not affect viability when the stimulus is of a different nature. In controls, there is no change in AEF present at baseline.

It is demonstrated that ER itself is not predictive of a drug effect, but baseline expression of AEF is predictive of a drug effect. Further, the conversion of AEF into an inactive state signals a biological event relevant to the receptor.

## Claims

1. An *in vitro* method for identifying a tumor treatable with an anti-estrogen, estrogen receptor antagonist or aromatase inhibitor, comprising:
a) exposing cancer cells or a tumor tissue specimen containing cancer cells obtained from a patient to an anti-estrogen receptor antibody under conditions appropriate for binding of the antibody to estrogen receptors in nuclei of the cancer cells; and
b) detecting presence or absence of focal binding of the antibody to estrogen receptors in the nuclei;
wherein the presence of focal binding indicates sensitivity of the tumor to treatment with the anti-estrogen, estrogen receptor antagonist or aromatase inhibitor and the absence of focal binding indicates lack of sensitivity of the tumor to treatment with the anti-estrogen, estrogen receptor antagonist or aromatase inhibitor.

2. The method of claim 1, further comprising detecting presence or absence of diffuse binding of the antibody to estrogen receptors in the nuclei.

3. The method of claim 1 or 2, wherein presence or absence of focal binding is detected by fluorescence or colorimetrically.

4. The method of any of claims 1-3, wherein presence or absence of focal binding is quantitated.

5. The method of claim 4, wherein presence or absence of focal binding is expressed as a percentage of activated ER foci (AEF)-positive cells.

6. The method of claim 4, wherein presence or absence of focal binding is expressed as a relative intensity or density of activated estrogen receptor foci.

7. A method for selecting drug treatment for a tumor, comprising:
a) exposing cancer cells or a tumor tissue specimen containing cancer cells obtained from a patient to an anti-estrogen receptor antibody under conditions appropriate for binding of the antibody to estrogen receptors in nuclei of the cancer cells;
b) detecting presence or absence of focal binding of the antibody to estrogen receptors in the nuclei; and
c) selecting an anti-estrogen, estrogen receptor antagonist or aromatase inhibitor for use in treating the tumor if focal binding is present.

8. The method of any of claims 1-7, wherein the degree of focal distribution of AEF in the cancer cells is 1-100%, 5-100%, 25-100% or 50-100%.

9. The method of any of claims 1-7, wherein an anti-estrogen, estrogen receptor antagonist or aromatase inhibitor is selected for use in treating the tumor.

10. An anti-estrogen, estrogen receptor antagonist or aromatase inhibitor for use in the treatment of a tumor previously identified as AEF positive wherein the AEF-positive status of the tumor is determined by;
a) exposing cancer cells or a tumor tissue specimen containing cancer cells obtained from a patient to an anti-estrogen receptor antibody under conditions appropriate for binding of the antibody to estrogen receptors in nuclei of the cancer cells; and
b) detecting presence or absence of focal binding of the antibody to estrogen receptors in the nuclei;
wherein the presence of focal binding indicates sensitivity of the tumor to treatment with the anti-estrogen, estrogen receptor antagonist or aromatase inhibitor and the absence of focal binding indicates lack of sensitivity of the tumor to treatment with the anti-estrogen, estrogen receptor antagonist or aromatase inhibitor.

11. An anti-estrogen, estrogen recentor antagonist or aromatase inhibitor for use according to claim 10, which is an ER-antagonist.

12. An anti-estrogen- estrogen recentor antagonist or aromatase inhibitor for use according to claim 10, which is an aromatase inhibitor.

13. A method for screening an antitumor drug or antitumor drug candidate for AEF inactivating activity which comprises:
a) providing cancer cells or a tumor tissue specimen containing cancer cells, wherein the cancer cells express a baseline degree of focal distribution of AEF and the AEF are detectably stained with an anti-estrogen receptor antibody;
b) exposing the cells or tissue specimen to the antitumor drug or antitumor drug candidate; and
c) detecting a decrease in the degree of focal distribution of the AEF relative to baseline as an indication of AEF inactivating activity of the antitumor drug or antitumor drug candidate, or detecting no substantial decrease in the degree of focal distribution of the AEF relative to baseline as an indication of lack of AEF inactivating activity of the antitumor drug or antitumor drug candidate.

14. The method or medical use of any of claims 1- 13, wherein the anti-estrogen receptor antibody is anti-ERα

## Patentansprüche

1. *In-vitro*-Verfahren zum Identifizieren eines Tumors, der mit einem Anti-Östrogen, einem Östrogenrezeptor-Antagonisten oder einem Aromatase-Inhibitor behandelt werden kann, das Folgendes beinhaltet:
a) Inkontaktbringen von Krebszellen oder einer von einem Patienten genommenen Krebszellen enthaltenden Tumorgewebeprobe mit einem Anti-ÖstrogenRezeptor-Antikörper unter Bedingungen, die zum Binden des Antikörpers an Östrogenrezeptoren in Kernen der Krebszellen geeignet sind; und
b) Erkennen der An- oder Abwesenheit einer fokalen Bindung des Antikörpers an Östrogenrezeptoren in den Kernen;
wobei die Anwesenheit einer fokalen Bindung Empfindlichkeit des Tumors für eine Behandlung mit dem Anti-Östrogen, Östrogenrezeptor-Antagonisten oder Aromatase-Inhibitor anzeigt und die Abwesenheit einer fokalen Bindung fehlende Empfindlichkeit des Tumors für eine Behandlung mit dem Anti-Östrogen, Östrogenrezeptor-Antagonisten oder Aromatase-Inhibitor anzeigt.

2. Verfahren nach Anspruch 1, das ferner das Erkennen der An- oder Abwesenheit einer diffusen Bindung des Antikörpers an Östrogenrezeptoren in den Kernen beinhaltet.

3. Verfahren nach Anspruch 1 oder 2, wobei die An- oder Abwesenheit einer fokalen Bindung durch Fluoreszenz oder kolorimetrisch erkannt wird.

4. Verfahren nach einem der Ansprüche 1-3, wobei die An- oder Abwesenheit einer fokalen Bindung quantifiziert wird.

5. Verfahren nach Anspruch 4, wobei die An- oder Abwesenheit einer fokalen Bindung als Prozentanteil von aktivierten ER-Foki-(AEF)-positiven Zellen ausgedrückt wird.

6. Verfahren nach Anspruch 4, wobei die An- oder Abwesenheit einer fokalen Bindung als relative Intensität oder Dichte von aktivierten Östrogenrezeptor-Foki ausgedrückt wird.

7. Verfahren zum Auswählen einer Arzneimittelbehandlung für einen Tumor, das Folgendes beinhaltet:
a) Inkontaktbringen von Krebszellen oder einer von einem Patienten genommenen Krebszellen enthaltenden Tumorgewebeprobe mit einem Anti-ÖstrogenRezeptor-Antikörper unter Bedingungen, die zum Binden des Antikörpers an Östrogenrezeptoren in Kernen der Krebszellen geeignet sind;
b) Erkennen der An- oder Abwesenheit einer fokalen Bindung des Antikörpers an Östrogenrezeptoren in den Kernen; und
c) Auswählen eines Anti-Östrogens, Östrogenrezeptor-Antagonisten oder Aromatase-Inhibitors zur Verwendung bei der Behandlung des Tumors, wenn eine fokale Bindung vorhanden ist.

8. Verfahren nach einem der Ansprüche 1-7, wobei der Grad an fokaler Verteilung von AEF in den Krebszellen 1-100 %, 5-100 %, 25-100 % oder 50-100 % beträgt.

9. Verfahren nach einem der Ansprüche 1-7, wobei ein Anti-Östrogen, Östrogenrezeptor-Antagonist oder Aromatase-Inhibitor zur Verwendung bei der Behandlung des Tumors ausgewählt wird.

10. Anti-Östrogen, Östrogenrezeptor-Antagonist oder Aromatase-Inhibitor zur Verwendung bei der Behandlung eines zuvor als AEF-positiv identifizierten Tumors, wobei der AEF-positiv-Status des Tumors bestimmt wird durch:
a) Inkontaktbringen von Krebszellen oder einer von einem Patienten genommenen Krebszellen enthaltenden Tumorgewebeprobe mit einem Anti-ÖstrogenRezeptor-Antikörper unter Bedingungen, die zum Binden des Antikörpers an Östrogenrezeptoren in Kernen der Krebszellen geeignet sind; und
b) Erkennen der An- oder Abwesenheit einer fokalen Bindung des Antikörpers an Östrogenrezeptoren in den Kernen;
wobei die Anwesenheit einer fokalen Bindung Empfindlichkeit des Tumors für eine Behandlung mit dem Anti-Östrogen, Östrogenrezeptor-Antagonisten oder Aromatase-Inhibitor anzeigt und die Abwesenheit von fokaler Bindung fehlende Empfindlichkeit des Tumors für eine Behandlung mit dem Anti-Östrogen, Östrogenrezeptor-Antagonisten oder Aromatase-Inhibitor anzeigt.

11. Anti-Östrogen, Östrogenrezeptor-Antagonist oder Aromatase-Inhibitor zur Verwendung nach Anspruch 10, das/der ein ER-Antagonist ist.

12. Anti-Östrogen, Östrogenrezeptor-Antagonist oder Aromatase-Inhibitor zur Verwendung nach Anspruch 10, das/der ein Aromatase-Inhibitor ist.

13. Verfahren zum Untersuchen eines Antitumor-Arzneimittels oder Antitumor-Arzneimittelkandidaten auf AEF inaktivierende Aktivität, das Folgendes beinhaltet:
a) Bereitstellen von Krebszellen oder einer Krebszellen enthaltenden Tumorgewebeprobe, wobei die Krebszellen einen Basisliniengrad an fokaler Verteilung von AEF exprimieren und die AEF erkennbar mit einem Anti-Östrogen-Rezeptorantikörper gefärbt sind;
b) Inkontaktbringen der Zellen oder der Gewebeprobe mit dem Antitumor-Arzneimittel oder dem Antitumor-Arzneimittelkandidaten; und
c) Erkennen einer Abnahme des Grades an fokaler Verteilung der AEF relativ zur Basislinie als Hinweis auf AEF inaktivierende Aktivität des Antitumor-Arzneimittels oder Antitumor-Arzneimittelkandidaten, oder Erkennen keiner erheblichen Abnahme des Grades an fokaler Verteilung der AEF relativ zur Basislinie als Hinweis auf eine fehlende AEF inaktivierende Aktivität des Antitumor-Arzneimittels oder Antitumor-Arzneimittelkandidaten.

14. Verfahren oder medizinische Verwendung nach einem der Ansprüche 1-13, wobei der Anti-Östrogen-Rezeptorantikörper Anti-ERα ist.

## Revendications

1. *Méthode in vitro* destinée à identifier une tumeur traitable par un anti-oestrogène, un antagoniste de récepteur d'oestrogène ou un inhibiteur d'aromatase, comprenant :
a) l'exposition de cellules cancéreuses ou d'un spécimen de tissu tumoral contenant des cellules cancéreuses, obtenus à partir d'un patient, à un anticorps anti-récepteur d'oestrogène dans des conditions appropriées pour la fixation de l'anticorps aux récepteurs d'oestrogène dans les noyaux des cellules cancéreuses ; et
b) la détection de la présence ou de l'absence de fixation focale de l'anticorps aux récepteurs d'oestrogène dans les noyaux ;
où la présence de fixation focale indique une sensibilité de la tumeur vis-à-vis d'un traitement par l'anti-oestrogène, l'antagoniste de récepteur d'oestrogène ou l'inhibiteur d'aromatase et l'absence de fixation focale indique un manque de sensibilité de la tumeur vis-à-vis d'un traitement par l'anti-oestrogène, l'antagoniste de récepteur d'oestrogène ou l'inhibiteur d'aromatase.

2. Méthode selon la revendication 1, comprenant en outre la détection de la présence ou de l'absence de fixation diffuse de l'anticorps à des récepteurs d'oestrogène dans les noyaux.

3. Méthode selon la revendication 1 ou 2, dans laquelle la présence ou l'absence de fixation focale est détectée par fluorescence ou par voie colorimétrique.

4. Méthode selon l'une quelconque des revendications 1-3, dans laquelle la présence ou l'absence de fixation focale est quantifiée.

5. Méthode selon la revendication 4, dans laquelle la présence ou l'absence de fixation focale est exprimée sous forme de pourcentage de cellules positives pour les foyers d'ER activés (AEF).

6. Méthode selon la revendication 4, dans laquelle la présence ou l'absence de fixation focale est exprimée sous forme d'intensité ou de densité relative de foyers de récepteurs d'oestrogène activés.

7. Méthode destinée à la sélection d'un traitement médicamenteux pour une tumeur, comprenant :
a) l'exposition de cellules cancéreuses ou d'un spécimen de tissu tumoral contenant des cellules cancéreuses, obtenus à partir d'un patient, à un anticorps anti-récepteur d'oestrogène dans des conditions appropriées pour la fixation de l'anticorps aux récepteurs d'oestrogène dans les noyaux des cellules cancéreuses ;
b) la détection de la présence ou de l'absence de fixation focale de l'anticorps aux récepteurs d'oestrogène dans les noyaux ; et
c) la sélection d'un anti-oestrogène, d'un antagoniste de récepteur d'oestrogène ou d'un inhibiteur d'aromatase pour une utilisation dans le traitement de la tumeur si une fixation focale est présente.

8. Méthode selon l'une quelconque des revendications 1-7, dans laquelle le degré de distribution focale des AEF dans les cellules cancéreuses est de 1-100%, 5-100%, 25-100% ou 50-100%.

9. Méthode selon l'une quelconque des revendications 1-7, dans laquelle un anti-oestrogène, un antagoniste de récepteur d'oestrogène ou un inhibiteur d'aromatase est sélectionné pour une utilisation dans le traitement de la tumeur.

10. Anti-oestrogène, antagoniste de récepteur d'oestrogène ou inhibiteur d'aromatase pour une utilisation dans le traitement d'une tumeur précédemment identifiée comme étant AEF-positive : où l'état AEF-positif de la tumeur est déterminé par :
a) l'exposition de cellules cancéreuses ou d'un spécimen de tissu tumoral contenant des cellules cancéreuses, obtenus à partir d'un patient, à un anticorps anti-récepteur d'oestrogène dans des conditions appropriées pour la fixation de l'anticorps aux récepteurs d'oestrogène dans les noyaux des cellules cancéreuses ; et
b) la détection de la présence ou de l'absence de fixation focale de l'anticorps aux récepteurs d'oestrogène dans les noyaux ;
où la présence de fixation focale indique une sensibilité de la tumeur vis-à-vis d'un traitement par l'anti-oestrogène, l'antagoniste de récepteur d'oestrogène ou l'inhibiteur d'aromatase et l'absence de fixation focale indique un manque de sensibilité de la tumeur vis-à-vis d'un traitement par l'anti-oestrogène, l'antagoniste de récepteur d'oestrogène ou l'inhibiteur d'aromatase.

11. Anti-oestrogène, antagoniste de récepteur d'oestrogène ou inhibiteur d'aromatase pour une utilisation selon la revendication 10, qui est un antagoniste d'ER.

12. Anti-oestrogène, antagoniste de récepteur d'oestrogène ou inhibiteur d'aromatase pour une utilisation selon la revendication 10, qui est un inhibiteur d'aromatase.

13. Méthode de criblage d'un médicament anti-tumoral ou d'un médicament anti-tumoral candidat pour une activité d'inactivation d'AEF, comprenant :
a) la fourniture de cellules cancéreuses ou d'un spécimen de tissu tumoral contenant des cellules cancéreuses, où les cellules cancéreuses expriment un degré de ligne de base de distribution focale d'AEF et les AEF sont colorés de manière détectable par un anticorps anti-récepteur d'oestrogène ;
b) l'exposition des cellules ou du spécimen de tissu au médicament anti-tumoral ou au médicament anti-tumoral candidat ; et
c) la détection d'une diminution du degré de distribution focale des AEF par rapport à la ligne de base comme indication d'une activité d'inactivation d'AEF du médicament anti-tumoral ou du médicament anti-tumoral candidat, ou la détection d'aucune diminution substantielle du degré de distribution focale des AEF par rapport à la ligne de base comme indication du manque d'activité d'inactivation d'AEF du médicament anti-tumoral ou du médicament anti-tumoral candidat.

14. Méthode ou utilisation médicale selon l'une quelconque des revendications 1-13, dans laquelle l'anticorps anti-récepteur d'oestrogène est l'anti-ERα.
